(19) Europäisches Patentamt
European Patent Office
Office européen des brevets

(11) Veröffentlichungsnummer: **0 163 222**
**B2**

(12) # NEUE EUROPÄISCHE PATENTSCHRIFT

(45) Veröffentlichungstag der neuen Patentschrift: **09.01.91**

(51) Int. Cl.$^5$: **C 07 C 237/06,** B 01 D 61/42

(21) Anmeldenummer: **85106133.3**

(22) Anmeldetag: **18.05.85**

(54) **Verfahren zur Gewinnung wässriger Carnitinamid-Lösungen.**

(30) Priorität: **26.05.84 DE 3419724**

(43) Veröffentlichungstag der Anmeldung:
**04.12.85 Patentblatt 85/49**

(45) Bekanntmachung des Hinweises auf die Patenterteilung:
**09.09.87 Patenblatt 87/37**

(45) Bekanntmachung des Hinweises auf die Entscheidung über den Einspruch:
**09.01.91 Patentblatt 91/02**

(84) Benannte Vertragsstaaten:
**BE CH DE FR GB IT LI NL**

(56) Entgegenhaltungen:
**DE-A-3 342 713**
**DE-B-1 090 676**
**DE-B-1 543 845**
**DE-C- 348 380**
**US-A-3 051 640**
**US-A-3 330 749**
**US-A-4 238 306**

(73) Patentinhaber: **BASF Aktiengesellschaft**
**Carl-Bosch-Strasse 38**
**D-6700 Ludwigshafen (DE)**

(72) Erfinder: **Puetter, Hermann, Dr.**
**Haardter Str. 1**
**D-6730 Neustadt (DE)**
Erfinder: **Roske, Eckhard**
**Hillensheimerstr. 4**
**D-6700 Ludwigshafen (DE)**
Erfinder: **Pander, Hans Joachim**
**Hochdorfer Str. 19**
**D-6701 Roedersheim-Gronau (DE)**

**EP 0 163 222 B2**

Courier Press, Leamington Spa, England.

**Beschreibung**

Diese Erfindung betrifft ein Verfahren zur Gewinnung weitgehend reiner wäßriger Carnitinamid-Lösungen durch Elektrodialyse roher, im wesentlichen Chlorid enthaltender wäßriger Lösungen dieser Verbindungen.

Das als Carnitin bezeichnete γ-Trimethylammonium-β-hydroxibutyrat der Formel

$$(CH_3)_3\overset{\oplus}{N}—CH_2—CHOH—CH_2—COO^{\ominus}$$

und seine Derivate, wie das Amid, sind gesuchte Pharmazeutika. Man stellt Carnitin z.B. nach dem in der DE—PS 1 090 676 beschriebenen Verfahren durch Hydrolyse von (3-Nitrilo-2-hydroxipropyl)-trimethylammoniumchlorid mit konzentrierter Salzsäure her. Das dafür benötigte Nitril gewinnt man z.B. dadurch, daß man Epichlorhydrin zunächst mit Cyanid und dann mit Trimethylamin (Ber. *86* (1953) 525; Z.physiol. Chem. 318 (1960) 129) oder in umgekehrter Reinehfolge umsetzt (s. US—PS 3,135,788).

Da diese Synthese mit einem Zwangsanfall erheblicher Mengen anorganischer Verbindungen, wie Salzsäure, Kochsalz und Ammoniumchlorid verbunden ist, und außerdem aufgrund mangelhafter Selektivität auch organische Verunreinigungen entstehen, ist die Isolierung der sehr gut wasserlöslichen Wertprodukte aus den erhaltenen wäßrien Lösungen außerordentlich schwierig. Weil an die Wertprodukte sehr hohe Reinheitsanforderungen gestellt werden und sich an die Synthese eine Racemattrennung anschließt, ist es erforderlich, die anorganischen Verbindungen und organischen Nebenprodukte möglichst weitgehend aus den rohen wäßrien Lösungen zu entfernen. Es wird außerdem verlangt, daß das Carnitin frei von Anteilen seiner Vorstufen Carnitinnitril und Carnitinamid ist und daß das Carnitinamid möglichst wenig Carnitin enthält.

Aufgabe der Erfindung war es, ein Verfahren zu finden, das es gestattet, möglichst reine wäßrige Lösungen von Carnitinamid aus den rohen wäßrigen Lösungen dieses Stoffes zu gewinnen, die anorganische Verbindungen und organische Nebenprodukte enthalten.

Es wurde nun gefunden, daß man weitgehend reine wäßrige Lösungen von Carnitinamid gewinnt, wenn man wäßrige Lösungen von Carnitinamid, die man durch Hydrolyse von Carnitinnitril mit Salzsäure erhalten hat, bei Temperaturen von −20°C bis +80°C elektrodialysiert.

Das neue Verfahren geht von solchen wäßrigen Lösungen von Carnitinamid aus, die bei der z.B. aus der DE—OS 14 43 847 bekannten Hydrolyse des Carnitinnitrils mit Salzsäure entstehen. Da bei der erfindungsgemäßen Elektrodialyse diese Ausgangslösungen eine so schnelle Verminderung der Salzsäurekonzentration eintritt, daß eine weitere Hydrolyse zum Carnitin, die sich üblicherweise bei der Hydrolyse von Carnitinnitril mit Salzsäure nicht verhindern läß, vermieden wird, ermöglicht das Verfahren dieser Erfindung eine besonders vorteilhafte Reindarstellung von Carnitinamid, das durch Hydrolyse von Carnitinnitril mit Salzsäure geweonnen wird, wobei eine hohe Selektivität erzielt wird. Hierbei ist es von Vorteil, wenn man bei der Hydrolyse von Carnitinnitril mit Salzsäure auf einen nicht vollständigen Umsatz hinarbeitet und solche wäßrigen Carnitinamid-Lösungen in die Elektrodialyse einbringt, die noch einen Restgehalt von 0,1 bis 10 Gew.-% an nicht hydrolysiertem Nitril aufweisen. Üblicheweie hat man bisher zur Vermeidung der unerwünschten Nebenreaktion die Hydrolyse durch Zugabe von Lösungsmitteln oder durch Neutralisation z.B. mit Natronlauge gestoppt, was besonders umständliche Aufarbeitungsmaßnahmen nötig machte, die nun nicht mehr erforderlich sind.

Die Elektrodialyse wird in an sich bekannten Elektrodialyseapparaten durchgeführt, wobei z.B. 1 bis 400 Zellenpaare eingesetzt werden können. Man elektrodialysiert bei Temperaturen von −20 bis +80°C, vorzugsweise bei 0 bis 45°C und einer Zellenspannung pro Teilzelle von 1 bis 2 Volt. Die Stromdichte beträgt z.B. 0,1 bis 10 $A/dm^2$, vorzugsweise 0,5 bis 3 $A/dm^2$. Die wäßrige hohe Carnitinamid-Lösung wird als Diluat eingesetzt. Als Konzentrat verwendet man z.B. eine wäßrige 0,1 bis 5%ige Kochsalzlösung. Die Anreicherung der Salzkonzentration im Konzentrat während des Betriebs gleicht man z.B. durch den Austausch von Konzentrat gegen Wasser aus. Als Elektrolyt verwendet man z.B. eine wäßrige 0,5 bis 15%ige Natriumsulfat-Lösung.

Nach dem erfindungsgemäßen Verfahren lassen sich die rohen wäßrigen Lösungen von Carnitinamid besonders vorteilhaft und ohne nennenswerten Velust an Wertprodukten reinigen. Dasd ist überraschend, da das Carnitinamid in den Lösungen als Kation vorliegt, so daß nicht auszuschließen war, daß auch das Carnitinamid durch die Elektrodialyse aus der Reaktionslösung entfernt wird.

## BEISPIEL

a) Herstellung von Carnitinnitril

$a_1$) 36,2 g (0,3 Mol) 3-Chlor-2-hydroxibutyronitril wurden in einem 300 ml-Rührautoklaven mit 118 g (2,0 Mol) wasserfreiem Trimethylamin unter Rühren bei 100°C und ca. 20 bar Eigendruck umgesetzt. Nach 24 Stunden wurde abgekühlt, der Trimethylaminüberschuß abgegast und das Kristallisat ausgetragen und getrocknet. Man erhielt 50,5 g eines trimethylaminhydrochloridfreien, NMR-reinen Carnitinnitrilchlorids (3-Nitrilo-2-hydroxipropyl-trimethylammoniumchlorid) = ca. 94,3% Ausbeute.

$a_2$) 239 g (2,0 Mol) 3-Chlor-2-hydroxibutyronitril wurden mit 442 g 40 %iger wäßriger Trimethylaminlösung bei 40°C innehalb von 5 h unter Rühren und schwachem Rückfluß umgesetzt. Der Rückflußkühler wurde mit einem Kühlmedium mit der Temperatur von −20°C betrieben. Die wäßrige

Reaktionslösung wurde im Rotationsverdampfer eingedampft, das Kristallisat abgetrennt und getrocknet. Es enthielt 12% $Me_3N.HCl$. Die Ausbeute betrug 83%, bezogen auf 3-Chlor-2-hydroxibutyronitril.

b) Elektrodialyse zur Herstellung einer reinen Carnitinamid-Lösung

Es wurde eine Elektrodialyseapparatur verwendet, die aus einer Zelle und drei Kreisläufen für den Elektrolyten, das Diluat und das Konzentrat bestand.

Jeder dieser Kreisläufe, der aus einer Magnetkreiselpumpe und einem gläsernen Schlangenkühlker mit aufgesetztem 2 1-Vorratskolben betand, war über Polyethylen-Schläuche mit der Zelle verbunden. Die Kreisläufe waren an einen Kryostaten angeschlossen. Die Zelle war mit zwei Elektroden von je 0,35 $dm^2$ geometrischer Fläche ausgerüstet (Anode: platiniertes Titanstreckmetall; Kathode: $V_2A$-Stahl), die Elektrodenräume waren von den angrenzenden Konzentraträumen durch ®Nafion-Kationenaustauschermembranen abgetrennt. Zwischen den Elektrodenräumen befanden sich 6 Konzentrat- und 5 Diluaträume in alternierender Anordnung, wobei die Räume voneinander abwechselnd durch ®Selemion AMV und Selemion CMV-Membranen getrennt waren. Alle Membranen hatten eine aktive Fläche von 0,37 $dm^2$. Sie wurden voneinander durch 2 mm dicke PVC-Rahmen getrennt, die mit Bohrungen für die Beströmung der Räume ausgerüstet waren. In den Rahmen befanden sich außerdem noch "Spacer", Lupolennetze, um den Abstand zwischen den Membranen zu gewährleisten. Als Elektrolyt wurde eine 5%ige wäßrige $Na_2SO_4$-Lösung, als Konzentrat eine 0,5%ige wäßrige NaC-Lösung und als Diluat eine rohe Carnitinamid-Lösung verwendet, die folgendermaßen hergesellt wurde: 25 g Carnitinnitrilchlorid, das entsprechend Absatz $a_1$ erhalten wurde, wurde in 50 ml 38%iger Salzsäure gelöst. Die Lösung wurde bei 25°C gerüht. In zeitlichen Abständen wurden der Lösung Proben entnommen und über $^{13}C$-NMR anaysiert (Tabelle 1).

TABELLE 1
Hydrolyse von Carnitinnitril

| | Carnitin-nitrilchlorid | Carnitin-amidchlorid | Carnitin |
|---|---|---|---|
| nach 23 h Reaktionsdauer: | 5% | 90% | 3% |
| nach 27 h Reaktionsdauer: | 1% | 94% | ca. 5% |

Vier wäßrige Reaktionslösungen aus dieser Umsetzung, die die aus der Tabelle 1, Zeile 1, ersichtliche Zusammensetzung aufwiesen, wurden in der oben baeschriebenen Apparatur bei Temperaturen von 0 bis 5°C und einer Stromstärke von maximal 2 A elektrodialysiert. Die Elektrodialyse wurde solange durchgeführt, bis eine Klemmenspannung von 30 V erreicht wurde.

Bei dieser Klemmenspannung wurde in einer Diluat/Konzentrat-Teilzelle eine Spannung von 1,5 V erreicht. Dann wurde die Elektrodialyse bei konstanter Klemmenspannung (30 V) und abfallender Stromstärke fortgesetzt, bid der pH-Wert im Diluat auf 2 angestiegen war. Die Ergebnisse der Elektrodialyse sind aus der Tabelle 2 zu ersehen:

TABELLE 2
Elektrodialyse von Carnitinamid

| | Diluateinsatz | | | | Diluataustrag | | | |
|---|---|---|---|---|---|---|---|---|
| Vers.Nr. | g | $Cl^{\ominus}$ (%) | $H^{\oplus}$ Mol/kg | Carnitin-amid-chlorid (g) | g | $Cl^{\ominus}$ (%) | $H^{\oplus}$ Mol/kg | Carnitin-amid-chlorid (g) |
| 1 | 219 | 32,2 | 7,3 | 59,5 | 247 | 3,7 | 0,17 | 46,8 |
| 2 | 192 | 32,1 | 7,4 | 51,2 | 167 | 5,0 | 0,22 | 42,2 |
| 3 | 204 | 31,4 | 7,3 | 57,3 | 157 | 6,0 | 0,21 | 48,8 |
| Gesamt | 615 | | | 168,0 | 571 | | | 137,8 |
| | | | | | | | | Ausbeute* 82,0% |

* Anmerkung: Die Ausbeute wurde ohne Berücksichtigung der Diluatreste in der Apparatur und von Proben bestimmt. Das Carnitinamidchlorid war 95%ig (NMR).

## Patentansprüche

1. Verfahren zur Gewinning weitgehend reiner wäßrier Lösungen von Carnitinamid, dadurch gekennzeichnet, daß man eine wäßrige Lösung von Carnitinamid, die man durch Hydrolyse von Carnitinnitril mit Salzsäure erhalten hat, bei Temperaturen von −20°C bis +80°C elektrodialysiert.

2. Verfahren nach Anspruch 1, dadurch gekennzeichnet, daß die wäßrige Ausgangslösung noch einen Restgehalt von 0,1 bis 10 Gew.% an nicht hydrolysiertem Carnitinnitril enthält.

3. Verfahren nach Anspruch 1, dadurch gekennzeichnet, daß die Zellenspannung pro Teilzelle 1 bis 2 Volt und die Stromdichte 0,1 bis 10 $A/dm^2$ beträgt.

## Revendications

1. Procédé pour l'obtention de solutions aqueuses très pures de carnitinamide, caractérisé en ce qu'on soumet une solution aqueuse de carnitinamide que l'on a obtenue par hydrolyse de carnitinonitrile et d'acide chlorhydrique à une électrodialyse à des températures de −20 à +80°C.

2. Procédé selon la revendication 1, caractérisé en ce que la solution aqueuse a encore une teneur résiduelle en carnitinonitrile non hydrolysé der 0,1 à 10% en poids.

3. Procédé selon la revendication 1, caractérisé en ce que la tension d'élément par cellule partielle s'élève à 1—2 V et la densité de courant à 0,1—10 $A/dm^2$.

## Claims

1. A process for obtaining a substantially pure aqueous solution of carnitinamide, wherein an aqueous carnitinamide solution which has been obtained by hydrolysis of carnitine nitrile with hydrochloric acid is subjected to electrodialysis at from −20 to +80°C.

2. A process as claimed in claim 1, wherein the aqueous starting solution still has a residual content of from 0.1 to 10% by weight of unhydrolyzed carnitine nitrile.

3. A process as claimed in claim 1, wherein the cell voltage per cell pair is from 1 to 2 volt, and the current density is from 0.1 to $A/dm^2$.